# EUROPEAN PATENT APPLICATION

(11) **EP 4 830 779 A1**
(43) Date of publication of application: **28.10.2026**
(21) Application number: 26174209.2
(22) Date of filing: 23.04.2026
(51) Int. Cl.: A61N 5/10

(54) **PORTABLE RADIATION ONCOLOGY SYSTEM**

(30) Priority: 24.04.2025 US 202563794259 P
(71) Applicant: Alliance Oncology, LLC, Plantation, Florida 33322 (US)
(72) Inventor: CHAMBERLAIN,, David, Reno (US)
(74) Representative: Dehns

(57) **Abstract**

A portable radiation oncology system is disclosed. The system includes a portable unit (100) configured to be placed on a level surface and relocated between treatment sites. The portable unit includes a treatment area (150) equipped with a medical radiation treatment facility (152), such as a medical linear particle accelerator, and a control console area positioned remotely from the treatment area. Internal shielding (148a) is disposed between the treatment area and the control console area to attenuate radiation leakage and scatter during operation. The system further includes external shielding (200) positioned outside the portable unit. The external shielding comprises a plurality of shielding barriers arranged to cover at least a rear side and opposing lateral sides of the treatment area. The system optionally includes cap shielding (300) positioned above at least a portion of the external shielding to attenuate upwardly directed radiation, particularly in environments with elevated nearby structures.

## Description

This application claims the benefit of, and priority to, U.S. Provisional Application No. 63/794,259, filed on April 24, 2025. This application is related to U.S. Application No. 16/802,402, filed on February 26, 2020, which is a continuation of U.S. Application No. 16/688,979, filed on November 19, 2019, which claims the benefit of, and priority to, U.S. Provisional Application No. 62/770,125, filed on November 20, 2018.

### BACKGROUND

### Field

This disclosure relates generally to portable radiation oncology system and more specifically to a portable radiation oncology system with internal and/or external shielding.

### Description of Related Art

A medical linear particle accelerator (LINAC) is widely used to treat cancer by using customized high energy x-rays or electrons to conform to a tumor's shape of a patient and destroy cancer cells while sparing surrounding normal tissue of the patient. A 6-8% rising cancer rate is driving demand for radiation therapy using LINACs. Modern LINACs improve treatment efficacy, while minimizing damage to surrounding healthy tissues.

Like all expensive equipment, a LINAC with normal usage (e.g., 25 treatments per day) would require regular maintenance in addition to daily or weekly-based calibrations. Typically, regular maintenance would require the LINAC to be shut down for a period of time that may take weeks or months. In addition, according to recent data, around 900 Linear accelerators in the U.S. are over 15 years old and require replacement. When an upgrade for a renovation or when new equipment installation is required, the LINAC is typically shut down. The shutdown of a single vault LINAC facility could cost over a million dollars of lost revenue during a multi-month shutdown.

There are existing mobile radiation oncology coach systems built to provide continuing services during regular maintenance, during an upgrade for renovation and/or when new equipment installation is required. Each of the existing mobile radiation oncology coach systems typically reside in a container truck having limited space, especially the height of the container. In addition, once the container truck arrives to a site, the elevation level of the container is much higher than the ground level, and stairs or patient lifts are required for access by people. In addition, the external shielding is not easily relocated or movable.

The inventors have recognized that there is a need for portable and/or interim (e.g., portable, substantially portable/movable, leasing) radiation oncology service solution that is capable of overcoming the foregoing shortcomings and maintaining high-quality care, referrals and revenue while the fixed site equipment is temporarily unavailable or where fixed site equipment are not possible.

### SUMMARY

Disclosed here are numerous aspects of a unique and advantageous portable radiation oncology system equipped with state-of-the-art LINAC system/facility that is able to provide the same or equivalent therapy/technology, such as accelerated treatment times, a six-point safety system, ergonomic operator controls and many patient-friendly features, that are typically offered to patients in leading cancer centers. While patients receive excellent clinical care, the user and/or owners of the portable radiation oncology experience no disruption in referrals, revenue or staffing during equipment upgrades or construction projects.

It should be understood that each of the foregoing and various aspects, together with those set forth in the claims and summarized above and/or otherwise disclosed herein, including the drawings, may be combined to support claims for a device, apparatus, system, method of manufacture, and/or use without limitation.

### BRIEF DESCRIPTION OF THE DRAWINGS

These and other features, aspects and advantages are described below with reference to the drawings, which are intended to illustrate, but not to limit, the invention. In the drawings, like reference characters denote corresponding features consistently throughout similar embodiments.
FIG. 1 depicts a front-right perspective view of an exemplary portable radiation oncology system having a portable unit with an external shielding in accordance with some embodiments. The portable radiation oncology system illustrated in FIG. 1 is in a clinic mode.
FIG. 2 is a higher elevational view of the exemplary portable radiation oncology system illustrated in FIG. 1.
FIG. 3 is a top view of the portable unit of the portable radiation oncology system illustrated in FIG. 1 without showing the external shielding.
FIG. 4 depicts a perspective view inside of a treatment room of the portable unit.
FIG. 5 depicts a perspective view inside of a control console room of the portable unit.
FIG. 6 depicts another perspective view inside of the control console room including a changing room and a restroom.
FIG. 7 is a top view of the portable unit illustrated in FIG. 3 with external shielding.
FIG. 8A depicts a side view showing the internal shielding located between the treatment room and the vestibule of the portable unit illustrated in FIG. 3. FIG. 8B is a cross-sessional view taken along A--A of FIG. 8A.
FIG. 9A depicts a side view showing the internal shielding located within a sliding door. FIG. 9B is a cross-sessional view taken along G--G of FIG. 9A.
FIG. 10A depicts a side view showing the internal shielding located between the vestibule and the control room of the portable unit illustrated in FIG. 3. FIG. 10B is a cross-sessional view taken along J--J of FIG. 10A.
FIGS. 11A-11F depict diagrams of radiation scatter and leakage measurements conducted on the exemplary portable radiation oncology system of FIG. 1 with no external shielding.
FIG. 12 is a top view of the portable unit illustrated in FIG. 3, showing an embodiment in which the external shielding is arranged in a configuration different from that illustrated in FIG. 7.
FIG. 13 is a top view of the portable unit illustrated in FIG. 3, showing an embodiment in which the external shielding is configured similarly to that illustrated in FIG. 12, and further includes cap shielding positioned above at least a portion of the external shielding.
FIG. 14 depicts a top perspective view of the portable unit illustrated in FIG. 3, showing the external shielding and the cap shielding in an assembled configuration.
FIG. 15 depicts a top perspective view of the external shielding with the cap shielding during a construction or installation phase.
FIG. 16 depicts an internal view of the external shielding with the cap shielding during construction or installation.
FIG. 17 depicts a top perspective view of the portable unit illustrated in FIG. 12, identifying a plurality of locations within the interior of the portable unit at which radiation leakage measurements are taken.
FIG. 18 depicts a top perspective view of the portable unit illustrated in FIG. 12, identifying a plurality of locations positioned outside the portable unit for radiation leakage measurements.
FIG. 19 depicts a plurality of measurement locations positioned further outward from the portable unit for evaluating radiation leakage levels at increasing distances from the system.

### DETAILED DESCRIPTION OF THE PREFERRED EMBODIMENT

As summarized above and illustrated in the drawings, disclosed herein are various aspects and alternative embodiments of a portable radiation oncology system.

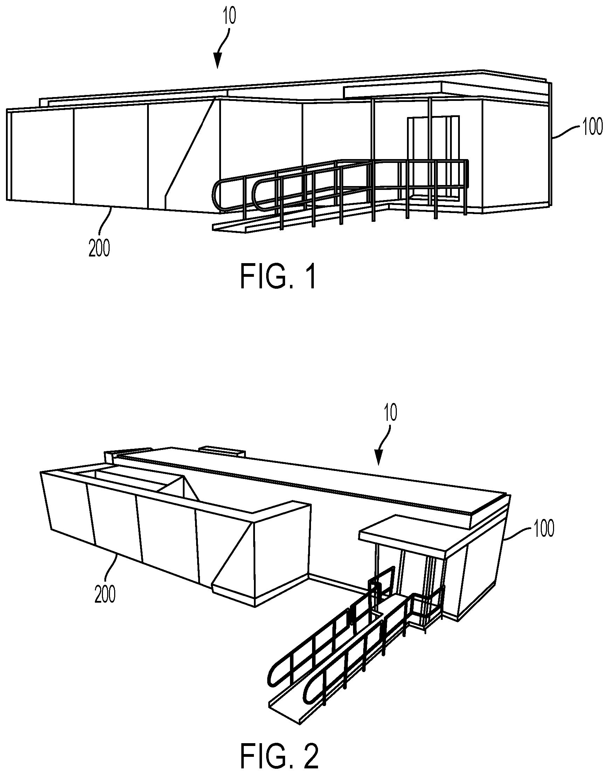

Referring to FIGS. 1-2, the exemplary portable radiation oncology system 10 described herein comprises a portable unit 100 and external shielding 200.

The portable unit 100 can be lifted, for example, by one or more heavy-duty lifts (not shown) on a flatbed trailer, which can be joined by a tractor or truck, during relocation (travel mode). When the portable unit 100 arrives at the designated site, the portable unit 100 can be detached from the trailer, for example, by one or more heavy-duty lifts so that the portable unit 100 is placed on a level area in a treatment configuration or clinical mode. The external shielding 200 is then optionally built around the portable unit 100 in specific configurations that will be described in detail hereafter.

In the exemplary embodiment, the portable unit 100 is about 58 feet in length, 12 feet in width, and about 11 feet in height. In the exemplary embodiment, the portable unit 100 has slide-out sections that allow the width of the portable unit 100 to be extended to a wingspan of about 16 feet in width when the portable unit 100 is in clinical mode. In some embodiments, the dimensions of the portable unit 100 are varied, including the dimensions of the slide-out sections.

When the portable unit 100 is situated at the designated site in clinical mode, the interior floor of the portable unit 100 can have the same level or higher than the ground level around the portable unit 100. In some embodiments, the interior floor of portable unit 100 is in the range of 0 to 3.5 feet higher than the ground level around the portable unit 100. In some embodiments, the interior floor of the portable unit 100 is in the range of 1 foot to 1.5 feet higher than the ground level around the portable unit 100. Preferably, the interior floor of the portable unit 100 is less than 1 foot higher than the ground level around the portable unit 100.

Preferably, the portable unit 100 is provided with sufficient area to be maneuvered and positioned for setup and takedown. The slide-out sections, entry ramp and any optional platform may require additional space on the passenger side of the portable unit 100. In some embodiments, access ramps and/or platforms are provided in alternative configurations.

Referring also to FIG. 2, proper and safe operation of the LINAC system can be obtained when the portable unit 100 is located on a substantially leveled area or firm pad. In some embodiments, the recommended level area has a dimension of, for example, 20 feet wide and 60 feet long.

Exemplary electrical options are provided below. In some embodiments, the electrical power for the portable unit 100 can be 480-volt AC, 3-phase Wye system with neutral and ground, at 200 Amperes. The frequency can be 60 +/- 2 Hertz. The maximum voltage variance can be +11% /-4% from nominal voltage. The maximum line regulation can be 2.5%. The maximum line-to-line imbalance can be 3%. For power cord/plug, a Russell Stoll 200 Amp plug can be supplied with the 50 feet power cord for connection to facility power. The cord connection point can be on the rear side of unit 100. For electrical support required by the facility, a 200 Amp, 480 Volt, 5 wire dedicated service including, for example, a Russell Stoll 200 Amp receptacle, can be mounted in a NEMA 3R rated enclosure to meet local codes requirements. An auxiliary earth ground connection point may be required in addition to the ground circuit within the pin and sleeve connector. An easily accessible NEMA 3R service disconnect in the immediate area is preferable.

The ground for the portable unit 100 can be, for example, originating at the system power source, e.g., transformer or a first access point of power into a facility, and continuous to the system power disconnect on the portable unit 100. This ground can be spliced with high compression fittings and can be terminated at each distribution panel it passes through. When it is broken for a connection to a panel, it can be connected into an approved grounding block with the incoming and outgoing ground in this same grounding block, which then can be connected to the steel panel. The connection at the power source can be at the grounding point of the neutral - ground if, for example, a Wye transformer is used. In the case of an external facility, it can be bonded or connected to the facility ground point at the service entrance.

When the portable unit 100 is generating radiation for either imaging or treatment, an exclusion zone is generally required to prevent exposure to either radiation workers or members of the public. This exclusion zone is generally determined based on the level of radiation exposure and local, state and federal requirements. It is possible to add shielding that allows a building to be closer, but the distances allowed may be determined by the customer's physical and local, state and federal requirements.

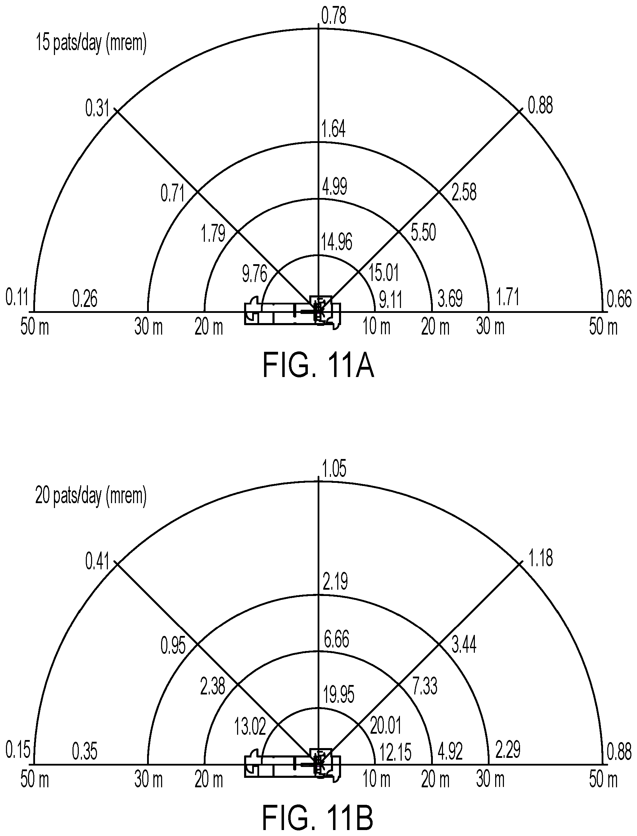

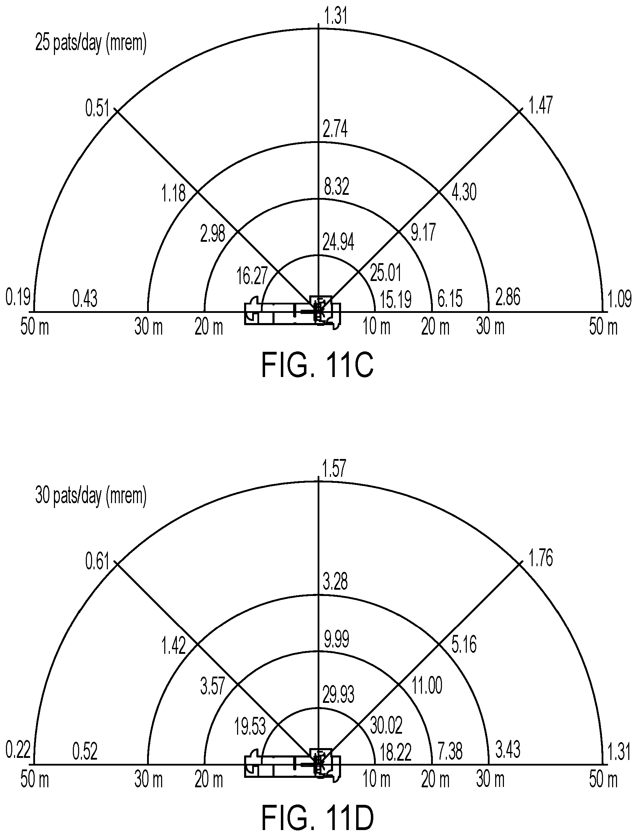

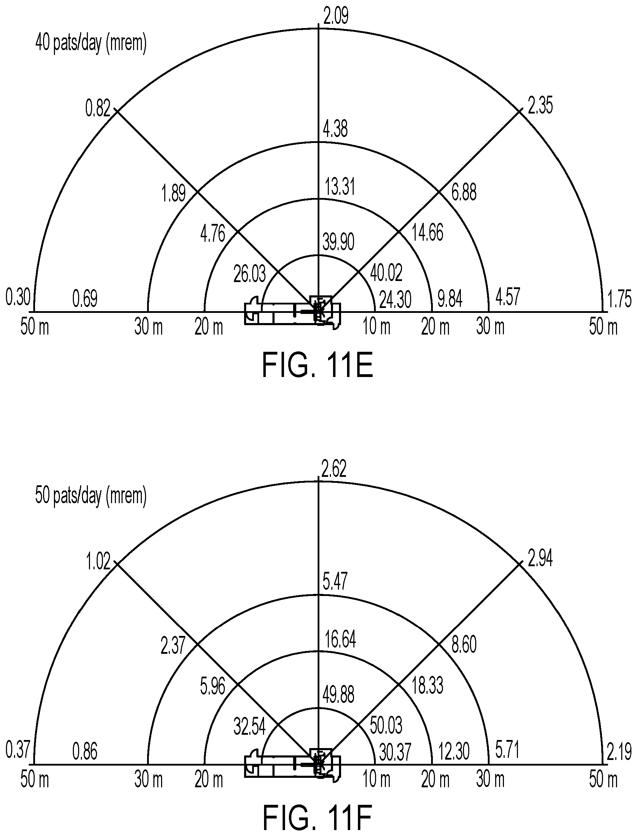

FIGS. 11A-11F depict diagrams of exemplary radiation scatter and leakage measurements conducted on the portable unit 100 of FIG. 1 with no external shielding. In some embodiments, measurements can be acquired by using a standard PTW Unidos E electrometer and a PTW TK-30 large volume chamber or optically stimulated luminescence dosimeters (OSLD) for measuring ionizing radiation exposure. Measurements can be engaged during clinical use over a 3-month period. All measurements can be displayed as equivalent doses in units of mrem. All displayed data are at the level of isocenter and radiate out at 10 m increments at various angles from zero degrees to 180 degrees. Although only one hemisphere is depicted in each of FIGS. 11A-11F, symmetry is assumed for all measurements. In addition, FIGS. 11A-11F have been scaled for different patient loads, or number of patients per day.

Based on the exemplary measurements and calculations, expected radiation exposure results for the portable unit 100 are provided in this disclosure and the above-mentioned distances are exemplary recommendations. The final site plan may also be determined based on distances to adjacent buildings and structures. In addition, with the installation of any ionizing radiation device, the appropriate site radiation survey should be conducted to verify compliance with these recommendations. Failure to correctly calculate and construct the radiation barriers and shielding as required may result in radiation exposure levels that are in excess of allowable limits and may present hazards to radiation workers and members of the public.

In some embodiments, the LINAC system includes a Varian Halcyon unit which uses a 6 MV FFF beam. The maximum dose rate can be 800 cGy/min. The maximum treatment field size can be 28 cm x 28 cm. The isocenter can be 100 cm. The unit can employ a beam stop so that the primary consideration for shielding is leakage and scatter. The Halcyon unit can deliver 3D, IMRT, and VMAT treatments.

Shielding considerations for the portable radiation oncology system 10 can have the following exemplary assumptions: workload, use factors (U = 1), occupancy times, design goal (permissible limits), distances, and utilization rate (beam on time). These considerations allow many variants to the external shielding design of the portable radiation oncology system 10. Design goals for unrestricted areas can be set as 1 mSv/yr (0.02 mSv/wk). Design goals for restricted areas can be set as 5 mSv/yr (0.10 mSv/wk). The conventional exposure rate in any one hour of 2 mR/hr guideline can be used. In addition, occupancy factors and utilization rate can be considered. In addition, actual instantaneous dose rates may optionally be considered as well.

Table 1 lists an exemplary series of iso-scatter/leakage measurements (see FIGS. 11A-11F) for calculating shielding requirements. As a guideline, parameters listed in Table 1 were used to calculate the required shielding.

**Table 1.**

| Parameters | Value |
|---|---|
| Workload | 1,200,000 mGy/wk (based on a workload of 40 patients per day) |
| Use Factor | 1.0 (leakage and scatter) |
| Occupancy | Based on location |
| Distances | Measured in meters with 0.3 meters from barrier |
| Design Goal | 1 mSv/year (unrestricted), 5 mSv/year (restricted) |
| IMRT | 4 (used for workload leakage) |

Table 2 lists a variety of works to be performed during radiation leakage measurements for the portable radiation oncology system. In some embodiments, the works include (1) crosscalibration of one or more survey meters to provide consistent dose-rate readings, (2) measuring dose rates at designated reference points inside the clinical treatment unit (e.g., outside of a patient vault) using multiple field sizes (including 28 cm x 28 cm, 10 cm x 10 cm) at cardinal gantry angles, (3) measuring dose rates at reference points outside the clinical treatment unit and outside external shielding walls, for example, at one foot from a shielding wall for a 28 cm x 28 cm field size at gantry angles of 0° and 270°, (4) performing skyshine radiation measurements at the same external points by identifying a maximum exposure as a function of increasing distance from the shielding wall (e.g., for a 28 cm x 28 cm field size), (5) measuring extended-distance points for selected configurations (e.g., 28 cm x 28 cm field size at gantry angle 270°), (6) measuring radiation levels in adjacent areas such as neighboring cancer-center spaces for selected configurations (e.g., 28 cm x 28 cm field size at gantry angle 270°), (7) calculating an approximate personnel exposure using a Varian formula based on the measurements to confirm the calculated value is below an applicable limit, and (8) declaring surrounding areas, including unrestricted public-access areas, safe based upon the measurement results.

**Table 2.**

| Exemplary Steps | Work Details |
|---|---|
| 1 | Cross calibration of survey meters. |
| 2 | Reference points inside the clinical treatment unit (outside of patient vault) measured with 28 cm x 28 cm and 10 cm x 10 cm field size at cardinal gantry angles. |
| 3 | Reference points outside the clinical treatment unit and outside the external shielding walls measured at one foot from the shielding wall for 28 cm x 28 cm field size and Gantry angles 0°/270°. |
| 4 | Skyshine radiation at the same external points outside the shielding walls measured by checking for the maximum exposure as a function of increasing distance from the shielding wall for 28 cm x 28 cm field size. |
| 5 | Extended distance points measured for 28 cm x 28 cm field size, Gantry angle 270°. |
| 6 | All areas inside neighboring cancer center measured for 28 cm x |
| | 28 cm field size and Gantry angle 270°. |
| 7 | Varian formula for determination of approximate exposure received by personnel for Halcyon testing calculated based upon measurements. Value is less than the limit. |
| 8 | Safety for all areas including surrounding areas of unrestricted access for the general public are declared safe based upon measurements. |

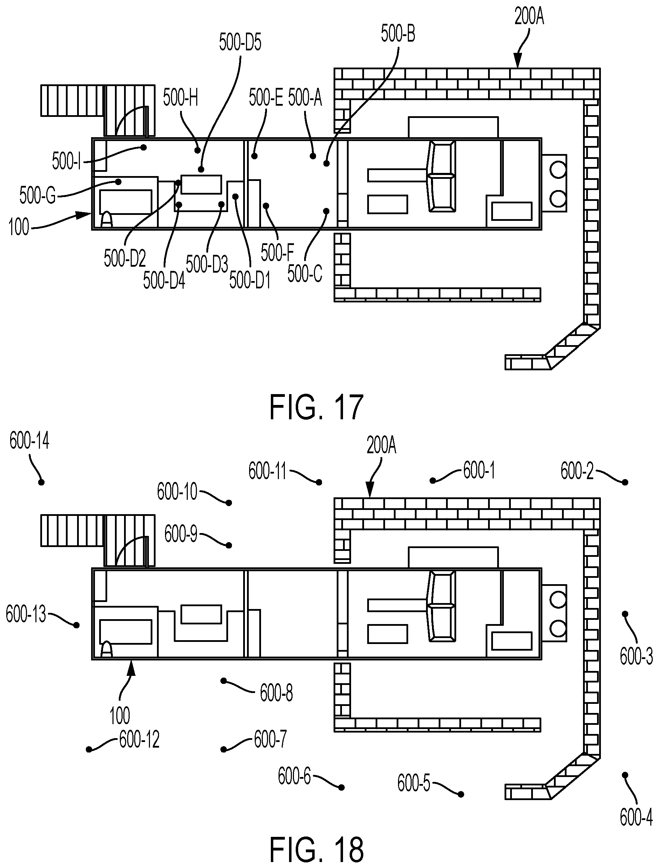

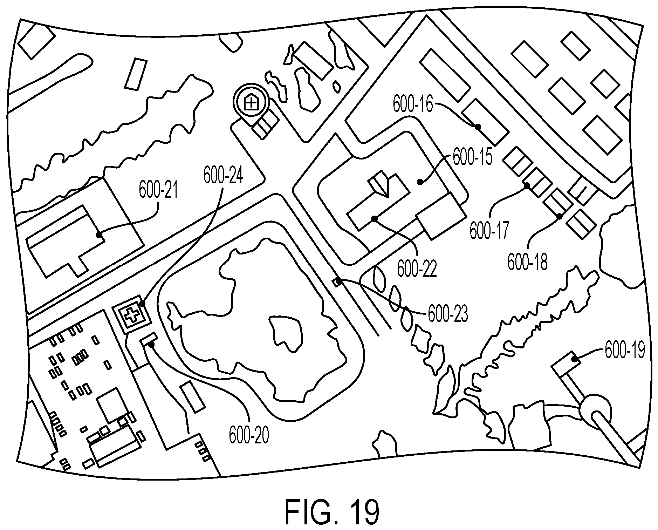

FIG. 17 shows a plurality of measurement locations within the interior of the portable unit 100 at which radiation leakage measurements are taken. FIGS. 18-19 show a plurality of measurement locations outside of the portable unit at which radiation leakage measurements are taken. In some embodiments, these locations correspond to reference points used during a radiation survey workflow (e.g., the radiation survey workflow summarized in Table 2), including interior reference points inside the clinical treatment unit (e.g., outside of a patient vault) measured using multiple field sizes (e.g., 28 cm x 28 cm, 10 cm x 10 cm) at cardinal gantry angles. In some embodiments, the locations further include exterior reference points outside the clinical treatment unit and outside external shielding walls, for example measured at 1 ft from the shielding wall at gantry angles of 0° and 270° for a 28 cm x 28 cm field size. In some embodiments, additional measurements are performed at the exterior reference points to evaluate atmospheric scatter (skyshine), for example by determining maximum exposure as a function of increasing distance from the shielding wall, and to evaluate extended-distance points and adjacent areas under selected field size and gantry-angle configurations. In some embodiments, the measurement results are used to calculate an estimated personnel exposure (e.g., using a Varian formula) and to verify that surrounding areas, including unrestricted public-access areas, satisfy applicable safety limits based on the measurements.

Tables 3 and 4 show measurement data within the interior of the portable unit 100 at locations labeled as reference numbers 500-A-500-I in FIG. 17. Unless otherwise noted, measurements are in mR/hr at 1 ft from surface. RTT locations (i.e., the physical positions or designated areas where the radiation therapist operates or interacts with the treatment system) are at counter edge in front of workstation locations. Table 2 has a field size of 10 cm x 10 cm and Table 3 has a field size of 28 cm x 28 cm, each has a collimator set at 45° and the dose rate 800 MU/min.

**Table 3.**

| Field Size 10 cm x 10 cm, Collimator 45°, Dose Rate 800 MU/min | | | | | |
|---|---|---|---|---|---|
| Location of Measurement | Location Number | Gantry 0° mR/hr | Gantry 90° mR/hr | Gantry 270° mR/hr | Gantry 180° mR/hr |
| Halcyon Electronics Cabinet | 500-A | 2.2 | 4.2 | 4.5 | 1.9 |
| Between Electronics and Door | 500-B | 1.5 | 2.5 | 2.6 | 1.2 |
| Vault Door in front of Left Side | 500-C | 4.0 | 6.8 | 7.5 | 3.4 |
| RTT space 1 | 500-D1 | 0.2 | 0.4 | 0.2 | 0.2 |
| RTT space 2 | 500-D2 | 0.5 | 1.0 | 0.7 | 0.4 |
| RTT space 3 | 500-D3 | 0.3 | 0.6 | 0.5 | 0.3 |
| RTT space 4 | 500-D4 | 0.4 | 1.0 | 0.8 | 0.3 |
| Middle of console floor | 500-D5 | 0.6 | 1.1 | 0.8 | 0.3 |
| Vestibule entrance | 500-E | 3.0 | 4.0 | 2.1 | 2.2 |
| Cabinets in Vestibule across from vault door | 500-F | 4.0 | 4.8 | 5.1 | 2.1 |
| Dressing room | 500-G | 0.4 | 0.6 | 0.7 | 0.3 |
| Wall between building entrance and vestibule | 500-H | 1.1 | 1.7 | 2.3 | 1.0 |
| Building entrance | 500-I | 0.9 | 0.9 | 1.0 | 0.8 |

**Table 4.**

| Field Size 28 cm x 28 cm, Collimator 45°, Dose Rate 800 MU/min | | | | | |
|---|---|---|---|---|---|
| Location of Measurement | Location Number | Gantry 0° mR/hr | Gantry 90° mR/hr | Gantry 270° mR/hr | Gantry 180° mR/hr |
| Halcyon Electronics Cabinet | 500-A | 13.0 | 23.0 | 20.0 | 15.5 |
| Between Electronics and Door | 500-B | 8.0 | 12.0 | 13.0 | 9.6 |
| Vault Door in front of Left Side | 500-C | 22.0 | 31.0 | 31.0 | 20.0 |
| RTT space 1 | 500-D1 | 1.1 | 1.7 | 1.4 | 1.1 |
| RTT space 2 | 500-D2 | 2.3 | 3.6 | 3.6 | 3.5 |
| RTT space 3 | 500-D3 | 1.2 | 2.1 | 2.7 | 1.7 |
| RTT space 4 | 500-D4 | 2.0 | 3.0 | 3.1 | 2.1 |
| Middle of console floor | 500-D5 | 2.4 | 3.9 | x | 2.9 |
| Vestibule entrance | 500-E | 10.5 | 23.0 | 20.0 | 16.1 |
| Cabinets in Vestibule across from vault door | 500-F | 16.0 | 24.0 | 30.0 | 17.7 |
| Dressing room | 500-G | 1.5 | 2.8 | 2.5 | 1.9 |
| Wall between building entrance and vestibule | 500-H | 5.5 | 9.7 | 8.4 | 7.3 |
| Building entrance | 500-I | 3.2 | 4.5 | 4.6 | 4.8 |

In some embodiments, a Varian Dose(x) estimate of total radiation exposure to Varian personnel during an installation procedure is determined, for example using Varian form L9330A Rev. 03 (page 3 of 3) for a Halcyon under an extreme condition (28 cm x 28 cm field size, collimator 45°), according to a weighted combination of measured dose rates at different gantry angles: 0°, 90°, 180°, 270°. The formula below can be used to calculate console-area maximum dose: Dose(x) = 3 hr × [0.7 × DR(G0) + 0.1 × DR(G90) + 0.1 × DR(G180) + 0.1 × DR(G270)], where DR(G0) is the measured dose rate at gantry angle 0°, DR(G90) is the measured dose rate at gantry angle 90°, DR(G180) is the measured dose rate at gantry angle 180°, and DR(G270) is the measured dose rate at gantry angle 270°.

This formula provides a conservative amount of time that is needed by engineers/workers to install the radiation machine. This amount of time (e.g., 3 hours) can be used to multiply the weighted combination of dose rates. For example, if DR(G0) is 2.4 mR/hr, DR(G90) is 6.8 mR/hr, DR(G180) is 3.5 mR/hr, DR(G270) is 3.6 mR/hr, the resulting dose (console-area maximum) is calculated as 9.2 mR. If the weighted combination of dose rates 3.07 mR/hr is taken without multiplying the time of 3 hours, the resulting weighted combination of dose rates of 3.07 mR/hr is way below the Varian limit of 20 mR/hr.

Tables 5-7 show measurement data outside of the portable unit at locations labeled by reference numbers 600-1, 600-2, 600-3, 600-4, 600-5, 600-6, 600-7, 600-8, 600-9, 600-10, 600-10a, 600-11, 600-12, 600-13, 600-14, 600-15, 600-16, 600-17, 600-18, 600-19, 600-20, 600-21, 600-22, 600-23, 600-24 in FIGS. 18-19. All measurement units are mR/hr at 1 foot from surface. RTT locations are at counter edge in front of workstation locations. Table 5 has a field size of 28 cm x 28 cm, Table 6 has a field size of 10 cm x 10 cm, and Table 7 has a field size of 4 cm x 4 cm, each has a collimator set at 45° and the dose rate 800 MU/min. Max point is at 1' off the wall unless otherwise stated.

**Table 5.**

| Field Size 28 cm x 28 cm, Collimator 45°, Dose Rate 800 MU/min | | | | | | | | |
|---|---|---|---|---|---|---|---|---|
| Location Number | G=0° mR/hr | G=90° mR/hr | G=180° mR/hr | G=270° mR/hr | Max Point | Distance (ft) | Far Point | Distance/ Location |
| 600-1 | 2.0 | 1.0 | 0.7 | 1.2 | 3.0 | 0.8 (tree) | 1.2 | max at halfway between fence and picnic table: 3.0 @180 |
| 600-2 | 1.1 | 2.5 | 1.6 | 1.3 | 2.6 | | 1.2 | max 180: 2.5/ 2.6 picnic table |
| 600-3 | 1.3 | 1.5 | 0.6 | 0.5 | 1.5 | | | |
| 600-4 | 1.1 | 1.5 | 1.4 | 1.4 | 1.5 | | | |
| 600-5 | 2.6 | 2.0 | 0.6 | 1.1 | 2.6 | | | |
| 600-6 | 0.5 | 2.9 | 1.8 | 2.0 | 2.9 | | | |
| 600-7 | 3.2 | 2.9 | 3.3 | 3.0 | 3.3 | | | |
| 600-8 | 4.2 | 2.7 | 3.0 | 3.5 | 4.2 | | | |
| 600-9 | 1.9 | 4.2 | 3.9 | 5.6 | 5.6 | | 1.2 | |
| 600-9a | 2.6 | 4.0 | 4.3 | 3.3 | 4.3 | | | |
| 600-10 | 1.6 | 2.2 | 2.0 | 2.0 | 2.2 | | 1.2 | |
| 600-10a | 1.9 | 2.3 | 2.0 | 2.0 | 2.3 | | | |
| 600-11 | | 0.8 | 0.3 | 0.4 | 0.8 | | 1.0 | |
| 600-12 | 2.0 | 2.5 | 2.8 | 3.0 | 3.0 | | 1.5 | Pad edge |
| 600-13 | 1.5 | 2.0 | 2.0 | 1.7 | 2.0 | | 1.0 | Pad edge |
| 600-14 | 1.7 | 2.3 | 2.4 | 1.9 | 2.4 | | 1.5 | Pad edge |
| 600-15 | 1.9 | 1.0 | 0.6 | 0.8 | 1.9 | | | Fence SE |
| 600-16 | 0.8 | 0.8 | 0.5 | 0.5 | 0.8 | | | Field SE 20' from fence |
| 600-17 | 1.0 | 0.6 | | | 1.0 | | | Field SE 50' from fence |
| 600-18 | 0.9 | | | | | | | Garbage Receptacle |
| 600-19 | 0.1 | | | | | | | |
| 600-20 | BG | | | | | | | |
| 600-21 | BG | | | | | | | |
| 600-22 | 0.5 | | | | | | | |
| 600-23 | 0.5 | | | | | | | |
| 600-24 | 0.4 | | | | | | | |

**Table 6.**

| Field Size 10 cm x 10 cm, Collimator 45°, Dose Rate 800 MU/min | | | | | | | | |
|---|---|---|---|---|---|---|---|---|
| Location Number | G=0° mR/hr | G=90° mR/hr | G=180° mR/hr | G=270° mR/hr | Max Point | Distance (ft) | Far Point | Distance/ Location |
| 600-1 | 0.6 | 0.3 | 0.6 | 1.1 | 1.1 | | | |
| 600-2 | 0.3 | 0.4 | 0.3 | 0.4 | 0.4 | | | |
| 600-3 | 0.8 | 0.4 | 1.2 | 0.4 | 1.2 | | | |
| 600-4 | 0.2 | 0.3 | 0.2 | 0.3 | 0.3 | | | |
| 600-5 | 0.6 | 2.0 | 1.7 | 0.4 | 2.0 | | | |
| 600-6 | 0.3 | 0.4 | 0.3 | 0.5 | 0.5 | | | |
| 600-7 | 0.5 | 0.6 | 0.4 | 0.6 | 0.6 | | | |
| 600-8 | 0.4 | 0.6 | 0.4 | 0.8 | 0.8 | | | |
| 600-9 | 0.6 | 0.9 | 0.6 | 1.1 | 1.1 | | | |
| 600-9a | 0.6 | 0.8 | 0.6 | 0.7 | 0.8 | | | |
| 600-10 | 0.4 | 0.5 | 0.3 | 0.5 | 0.5 | | | |
| 600-10a | 0.3 | 0.5 | 0.3 | 0.4 | 0.5 | | | |
| 600-11 | | 0.1 | | 0.1 | | | | |
| 600-12 | 0.4 | 0.5 | 0.3 | 0.6 | 0.6 | | | |
| 600-13 | 0.3 | 0.4 | 0.3 | 0.4 | 0.4 | | | |
| 600-14 | 0.3 | 0.5 | 0.3 | 0.4 | 0.5 | | | |
| 600-15 | | 0.4 | | 0.2 | | | | Fence SE |
| 600-16 | | 0.2 | | 0.2 | | | | Field SE 20' from fence |
| 600-17 | | | | | | | | Field SE 50' from fence |
| 600-18 | | | | | | | | |
| 600-19 | | | | | Houses now at this location; measurement is in Cul-de-sac | | | |
| 600-20 | | | | | | | | |
| 600-21 | | | | | | | | |
| 600-22 | | | | | | | | |
| 600-23 | | | | | | | | |
| 600-24 | | | | | | | | |

**Table 7.**

| Field Size 4 cm x 4 cm, Collimator 45°, Dose Rate 800 MU/min | | | | | | | | |
|---|---|---|---|---|---|---|---|---|
| Location Number | G=0° mR/hr | G=90° mR/hr | G=180° mR/hr | G=270° mR/hr | Max Point | Distance (ft) | Far Point | Distance/ Location |
| 600-1 | 2.1 | 0.2 | 0.5 | 1.7 | 2.1 | | | |
| 600-2 | 1.7 | 0.1 | 0.1 | 0.2 | 1.7 | | | |
| 600-3 | 1.9 | 0.4 | 0.7 | 0.3 | 1.9 | | | |
| 600-4 | 1.4 | 0.1 | 0.0 | 0.2 | 1.4 | | | |
| 600-5 | 3.5 | 1.5 | 1.4 | 0.2 | 3.5 | | | |
| 600-6 | 0.2 | 0.3 | 0.1 | 0.1 | 0.3 | | | |
| 600-7 | 0.2 | 0.2 | 0.1 | 0.2 | 0.2 | | | |
| 600-8 | 0.2 | 0.3 | 0.1 | 0.3 | 0.3 | | | |
| 600-9 | 0.3 | 0.4 | 0.1 | 0.4 | 0.4 | | | |
| 600-9a | 0.3 | 0.2 | 0.1 | 0.4 | 0.4 | | | |
| 600-10 | 0.1 | 0.1 | 0.1 | 0.2 | 0.2 | | | |
| 600-10a | 0.2 | 0.1 | 0.1 | 0.1 | 0.2 | | | |
| 600-11 | | 0.1 | | 0.1 | | | | |
| 600-12 | 0.2 | 0.2 | 0.1 | 0.2 | 0.2 | | | |
| 600-13 | 0.2 | 0.1 | 0.0 | 0.2 | 0.2 | | | |
| 600-14 | 0.2 | 0.1 | 0.0 | 0.2 | 0.2 | | | |
| 600-15 | | 0.5 | | 0.6 | | | | Fence SE |
| 600-16 | | 0.3 | | 0.1 | | | | Field SE 20' from fence |
| 600-17 | | | | | | | | Field SE 50' from fence |
| 600-18 | | | | | | | | Garbage Receptacle |
| 600-19 | | | | | Houses now at this location; measurement is in Cul-de-sac | | | |
| 600-20 | | | | | | | | |
| 600-21 | | | | | | | | |
| 600-22 | | | | | | | | |
| 600-23 | | | | | | | | |
| 600-24 | | | | | | | | |

The data obtained from radiation measurements demonstrates the effectiveness of the disclosed shielding design. Measurements were conducted under worst-case operating conditions, including use of the largest available radiation field size, as well as under more representative clinical field sizes, to evaluate shielding performance across a range of operating scenarios. Radiation measurements were collected at multiple locations surrounding the structure, thereby providing a comprehensive assessment of radiation leakage and scatter. Ongoing personnel monitoring further confirms the effectiveness of the shielding configuration and supports the suitability of the system for providing radiation therapy services in environments such as rural communities, hospitals, and clinics requiring temporary or supplemental treatment capacity.

By way of example, using a highest measured radiation level of approximately 5.6 mR/hr outside the enclosure, and assuming radiation generation for approximately one hour per day, five days per week, and twelve months per year, the resulting annual exposure at that location would be approximately 1,344 mR. This value is below the maximum occupational exposure limit of 5,000 mR per year. Moreover, when occupancy factors and the limited duration of presence at such locations are considered, actual exposure levels are further reduced. This calculation reflects a worst-case scenario using the largest field size. When a smaller, more representative field size of approximately 10 cm is used, the maximum measured exposure decreases by approximately a factor of five, resulting in an annual exposure of less than approximately 300 mR. Additional separation distance, such as that provided by perimeter fencing to restrict access, further reduces exposure levels to well below applicable limits for both occupational workers and members of the public.

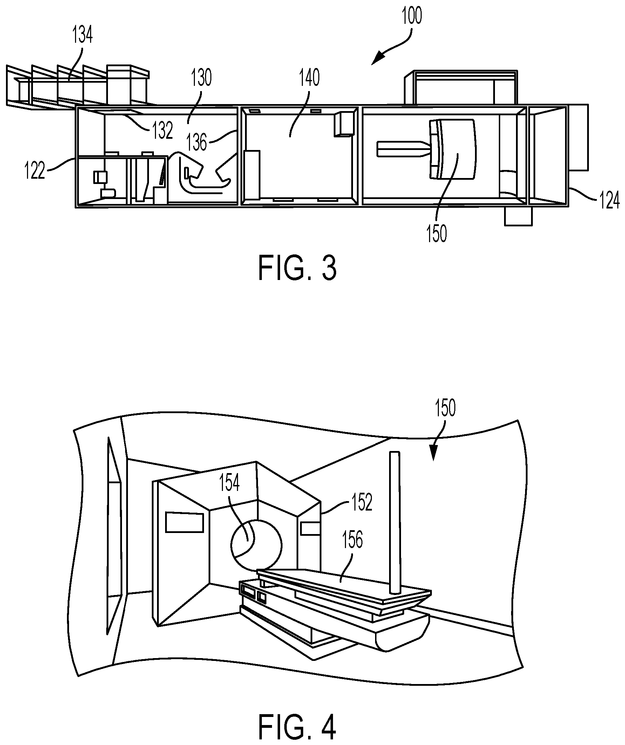

Referring to FIG. 3, the portable unit 100 has a front end 122 and a rear end 124. Between the front end 122 and rear end 124, the interior space of the unit 100 can be partitioned into multiple rooms. In some embodiments, as shown in FIG. 3, unit 100 includes a control console room or area 130, a vestibule 140, and a treatment room or area 150. As shown in FIG. 3, treatment room 150 is closer to the rear end 124 than the vestibule 140 and the control console room 130 while the vestibule 140 is closer to the rear end 124 than the control console room 130. In some embodiments, other partitions or arrangements for the rooms/areas 130, 140, 150 can be provided. For example, in some embodiments, only the treatment room 150 and control console area 130 are partitioned. The control console room 130 can also be an entry room and has a front door 132 for entering and exiting unit 100. A ramp 134 can be provided to facilitate access to unit 100.

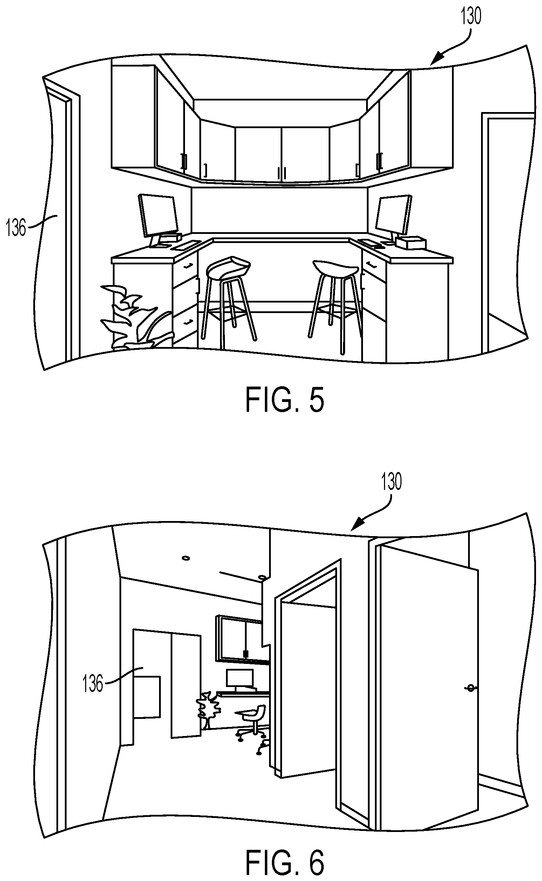

Referring also to FIGS. 5-6, in some embodiments, the control console room 130 can contain the operator's station, and/or scheduling computers. As depicted in FIG. 6, the control console area 130 can be partitioned with a changing room and a restroom, which can be optional. In some embodiments, the control console area 130 has an access door 136 for connecting the control console area 130 and the vestibule 140.

The unit 100 provides shielding for the control console area 130. Lead (Pb) shielding is used in the walls and doors to block line of site leakage and scatter. During treatment, no occupancy is allowed in the vestibule/entry room.

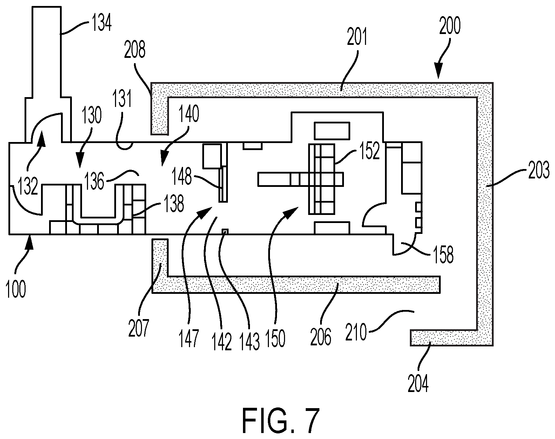

Referring also to FIG. 7, in some embodiments, door 142, for example a sliding door, is provided for connecting the vestibule 140 and the treatment room 150. The sliding door 142 can slide into wall 148. The sliding door 142 can be loaded with lead bricks to form a pocket door. In one embodiment, the pocket door 142 can weigh about 5000 lbs (2268 kg). In one embodiment, the sliding door 142 requires a separate partial wall/nub 143 for mating or overlapping with sliding door 142 to be closed properly and/or securely.

A patient under treatment can also enter unit 100 from the front door 132. After confirming by a representative of the control console room 130, the patient can enter the vestibule 140 through the access door 136. Then the patient can be guided to the treatment room 150 for treatment through the pocket door 142.

Referring also to FIG. 4, in some embodiments, the treatment room 150 can be designed for installation of a LINAC system 152 or other treatment or diagnostic instrument(s). A LINAC system 152 generally uses microwave technology to accelerate electrons in a wave guide and enable these electrons to collide with a heavy metal target to produce high-energy x-rays. These high energy x-rays can be shaped as they exit the machine to conform to the shape of the patient's tumor, enabling the customized beam to be directed to the patient's tumor. In some embodiments, the x-ray beam comes out of a part of the accelerator called a gantry 154, which can be rotated around the patient. Radiation can be delivered to the tumor from many angles by rotating the gantry 154 and moving the treatment couch 156.

In some embodiments, to ensure a safe environment with respect to radiation exposure in the vicinity of the unit 100, when the system is generating radiation for either imaging or treatment, an exclusion zone is provided to prevent exposure to either radiation workers or members of the public. This exclusion zone can be determined based on the level of radiation exposure and local, state, and federal requirements. It is possible to add shielding that allows a building to be closer, but the actual distances allowed will be determined by the customer's physicist and local, state, and federal requirements.

In some embodiments, expected radiation exposure results for unit 100 are provided based on shielding calculations. It is understandable that it is the responsibility of the user, in conjunction with their Safety Officer and local, state or federal regulations, to determine the final site plan and allowable distances to adjacent buildings and structures. In addition, as with the installation of any ionizing radiation device, the user is responsible for conducting the appropriate site radiation survey to verify compliance with these applicable requirements. Failure to correctly calculate and construct the radiation barriers and shielding as required may result in radiation exposure levels that are in excess of allowable limits and may present inappropriate hazards to radiation workers and members of the public.

Because radiation may scatter or leak from the treatment room 150 during a patient's treatment, protection to people outside of the treatment room 150 is desired. In some embodiments, the unit 100 can have shielding for protecting the control console area 130 during treatment operations. In some embodiments, lead (Pb) shielding, for example interlocked lead bricks, is used in walls 138, 148, 143 and door 142 to block line of site of leakage and scatter. In other embodiments, standard shielding materials can be used. The wall 138 between the vestibule 140 and the control console room 130 can provide secondary or additional shielding for protecting the control console area 130.

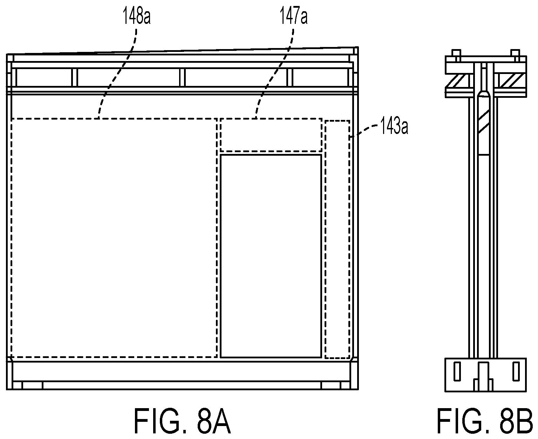

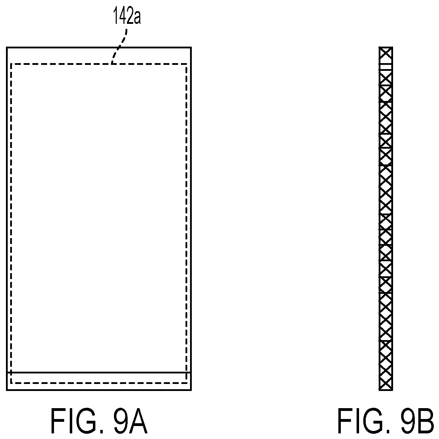

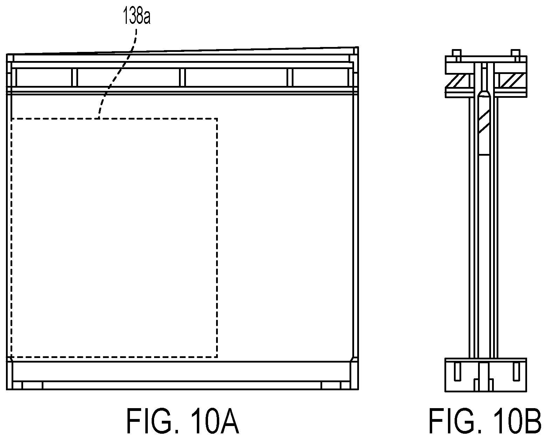

As shown in FIG. 8A, internal shielding 148a is installed on wall 148, internal shielding 143a is installed in partial wall/nub 143, and internal shielding 147a is installed on the top portion of the doorway 147. As shown in FIG. 3, wall 148, wall/nub 143 and doorway 142 are located between the treatment room 150 and the vestibule 140. Referring also to FIG. 9A, internal shielding 142a is installed on the pocket door 142 between the vestibule 140 and the treatment room 150. As shown in FIG. 10A, internal shielding 138a is installed on wall 138 between the control console room 130 and the vestibule 140.

In some embodiments, using standard lead (Pb) shielding materials, the thickness of lead bricks on the interior wall 138 between the control console room 130 and the vestibule 140 is 2 inches (5.08 cm), the thickness of lead bricks on the interior wall 148 between the vestibule 140 and the treatment room 150 is 4 inches (10.16 cm), the thickness of lead bricks on the pocket door is 2 inches (5.08 cm), with all walls having a minimum of ½ inches (1.27 cm) steel plates. In some embodiments, the access door 136 between the control console room 130 and the vestibule 140 can be made without shielding and is there for privacy purposes only or omitted entirely. In some embodiments, different thicknesses of the shielding can be used based on different shielding materials and/or radiation scatter and/or leakage. In some embodiments, the access door 136 does not require a separate partial wall/nub 131 for mating or overlapping with access door 136 to be closed properly and/or securely. In some embodiments, the portable radiation oncology system 10 does not require external shielding as a result of the internal shielding and/or the lack of direct line of sight from the treatment area to the outside of the system.

In some embodiments, the pocket door 142 between the vestibule 140 and the treatment room 150 can be a steel door with 2 inches (5.08 cm) of lead bricks. The steel plates holding the lead in place can be ¼ inch (0.635 cm) steel or ½ inch (1.27 cm) steel.

In some embodiments, the unit 100 advantageously incorporates alternating doors between the treatment room 150, vestibule 140 and control console room 130 to provide effective shielding. In some embodiments, non-manual or automated doors may optionally be used.

In some embodiments, unit 100 is equipped with limited yet adequate internal shielding to achieve energy efficiency for relocation of unit 100 to a designated site. The protection of the public external to the unit 100 is achieved using external shielding provided on the outside of unit 100.

In some embodiments, concrete walls 201, 203, 204, 206, 207, 208 (altogether 200) made of standard density concrete for the external shielding are used surrounding unit 100. The amount of external shielding required can be dependent on, for example, one or more or all of the following factors: workload, distance to surrounding areas, occupancy of surrounding areas, height of surrounding buildings, density of concrete used for shielding, and/or barrier location. The external shielding may be customized and does not need to be symmetric depending on the above-listed parameters. The closer unit 100 is placed to an existing occupied structure, the more shielding will generally be required.

In some embodiments, the concrete walls 201, 203, 206 are placed 3 feet from the unit. As shown in FIG. 7, concrete walls 208, 201, 203, 204 are connected in series and seamlessly with wall 208 substantially adjacent to unit 100 at the vestibule 140 near the console room 130. Concrete walls 207, 206 are connected in series and seamlessly with wall 207 substantially adjacent to unit 100 at the vestibule 140 near the console room 130. As illustrated in FIG. 7, concrete walls 201, 203, 204, 206, 207, 208 altogether cover three entire sides of the treatment area 150. In some embodiments, the concrete walls are formed in opposing "L" shapes. In some embodiments, the concrete walls are in opposing "L" and "J" shapes to create passageways for entry and/or exit. In some embodiments, the concrete walls range in lengths of 35 - 50 feet, or in lengths of 35-45 feet. In some embodiments, the concrete walls range in widths of 5-35 feet, depending on the location. These measures correspond to a portable unit or trailer of dimensions the portable unit 100 of about 45-65 feet in length, 12-18 feet in width, and about 12 feet in height.

As depicted in FIG. 7, a placement of the location of the external shielding 200 in relation to the LINAC system 152 is also shown. One advantage of the placement is that there is no requirement for any external wing walls (i.e., walls separated from the main external shielding structure) to take away any solid angle scatter or leakage radiation from exiting to the front end of the unit where the patients would enter the unit. The placement shown in FIG. 7 does not have any access (e.g., a wheelchair lift) at the middle of the unit. As such, the main external shielding structure can expand closer to the internal console wall 138. Considering the 4" leaded wall 148 adjacent to the treatment room 150 and moving the external shielding towards the front of the unit, allows flexibility to not have to install external shielding right up against the structure, but also cuts off any solid angle scatter from the treatment machine reaching the front of the unit. This will allow better patient flow and patient safety. It also eliminates the need for any wing walls.

In some embodiments, passageway 210 can be created between walls 204, 206 for an emergency exit from the treatment room 150 through door 158.

The height of the walls 200 is dependent on location of nearby structures. This will be different in each location where the unit is placed. The height of the external barriers must block a direct line of sight of the leakage coming from any gantry position.

In some typical examples, the portable radiation oncology system workload stays fairly constant (35-40 patients per day). The radiation decreases by 1/R², where R is the distance from the radiation source. In other words, when the distance from the radiation source is doubled, the radiation exposure decreases by approximately ¼. Occupancy Rate (T) can be determined by how often someone will be in a certain area. If people are in an area 100% of the time (T = 1) the machine is on, then that area must be shielded as needed/appropriately. If a person is in an area where there is little to no occupancy and no direct line of sight to the particle accelerator, that area would not need as much shielding. If there are multi-story structures, then that can be taken into account as well in determining the appropriate shielding.

In some embodiments, the external shielding recommendation is based on Table 7 below.

**Table 7.**

| Parameters | Value |
|---|---|
| Design Goal | 0.02 mGy/wk (unrestricted) |
| Workload | 1,200,000 mGy/wk |
| Leakage | 0.05 % |
| Occupancy Rate | T = 0.5 and T = 1.0 |
| Shielding | Normal Density Concrete (147 pcf) |

For the shielding parameter, in some embodiments, concrete of around 147 pcf is used to achieve more cost-effective result for adequate shielding.

FIG. 7 depicts a top view of the portable unit 100 with external shielding 200 positioned according to some embodiments. The unit 100 illustrated in FIG. 7 is in a clinic mode. As shown in FIG. 7, each of the external shielding barriers or walls 200 is placed at a calculated and/or predetermined distance from the nearest surface of unit 100. In some embodiments, the calculated distance is 3 meters. In some embodiments, at least 1 meter is disposed between the furthest out wall and the shielding to account for room to maneuver around the unit when parked.

In some embodiments, the height of the external shielding walls 200 is selected based on site-specific conditions, such as the proximity, elevation, and occupancy of nearby buildings or other structures. Accordingly, the wall height may vary from one installation location to another. The external barriers 201, 203, 204, 206, 207, and 208 can be sized and arranged such that, for any gantry position, there is no direct line of sight between a potential leakage source associated with the gantry and areas outside the external shielding. In this manner, the barriers help mitigate radiation leakage and/or scatter that could otherwise propagate above the wall height. In alternative embodiments, comparable shielding performance can be achieved using different barrier configurations, including alternative thicknesses, heights, materials, and/or combinations thereof, depending on applicable design goals and site constraints.

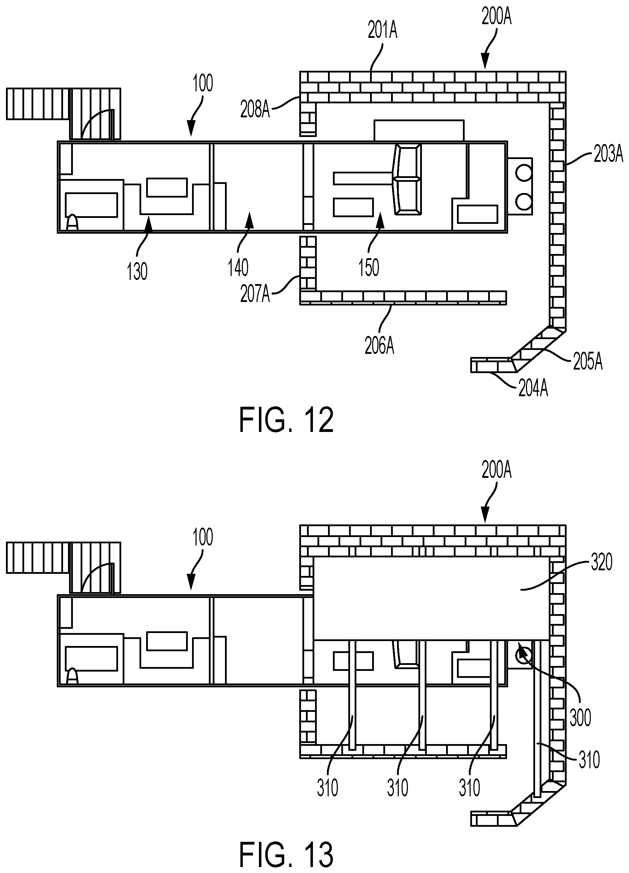

FIG. 12 illustrates a top view of the portable unit 100 with an alternative external shielding configuration 200A that differs from the arrangement depicted in FIG. 7. In this embodiment, the external shielding 200A comprises a plurality of concrete walls 208A, 201A, 203A, 205A, and 204A that are connected in series and arranged in a substantially continuous and seamless manner. The wall 208A is positioned substantially adjacent to the boundary between the vestibule 140 and the treatment area 150, thereby providing shielding coverage proximate to the radiation source while minimizing unnecessary extension of shielding structures into lower-exposure regions of the portable unit. Similarly, concrete walls 207A and 206A are connected in series and seamlessly arranged, with wall 207A also positioned substantially adjacent to the border between the vestibule 140 and the treatment area 150.

In contrast to the external shielding configuration shown in FIG. 7, a principal distinguishing feature of the external shielding 200A is that walls 208A and 207A are shifted or "pushed back" toward the treatment area 150. By relocating these shielding barriers closer to the source of radiation emission, the overall volume and quantity of shielding material required to achieve acceptable radiation attenuation levels may be reduced. This configuration allows for a reduction in material usage, installation time, and overall construction cost of the external shielding 200A, while still maintaining shielding performance comparable to that of the previously described external shielding arrangements.

Another distinguishing feature of the external shielding 200A is the inclusion of an additional concrete wall 205A positioned between walls 203A and 204A. In this arrangement, wall 205A is oriented to such that walls 203A and 205A form an angle of substantially 135 degrees, and walls 204A and 205A form another angle of substantially 135 degrees. This angled configuration is configured to intercept and attenuate scatter radiation more efficiently than linear wall arrangements, while also enabling a more compact shielding footprint and a smooth passageway.

Except as expressly described above, other structural, dimensional, material, and functional features of the external shielding 200A may be similar or identical to those of the external shielding 200 previously described and accordingly are not repeated herein for the sake of brevity.

FIG. 13 is a top view of the portable unit 100 configured with external shielding 200A and further optionally including a cap shielding 300. In some embodiments, the cap shielding 300 is constructed and positioned to attenuate radiation that may be emitted upwardly from the top side of the portable unit 100 during operation of the medical treatment facility. Such upward radiation attenuation may be particularly desirable in situations where nearby structures, such as tall commercial buildings, residential buildings, or other elevated occupied areas, are located in proximity to the site at which the portable unit 100 is deployed.

In some embodiments, the cap shielding 300 includes supporting frames 310 in the form of beams. A roof 320 can be mounted to the supporting frames 310. Preferably, when the portable unit 100 is positioned as shown in FIG. 13, each of the supporting frames 310 extends laterally across a top portion of the external shielding walls 200A along a direction substantially perpendicular to a longitudinal direction of the portable unit 100.

In the embodiment illustrated in FIG. 13, the cap shielding 300 is positioned on top of the external shielding 200A and configured to cover only a portion of the footprint defined by the external shielding 200A. By locating the cap shielding 300 above selected regions of the external shielding 200A, the system can selectively block potential radiation leakage paths that extend above the height of the external shielding walls, while limiting the overall size, weight, and material usage of the cap shielding structure to reduce cost.

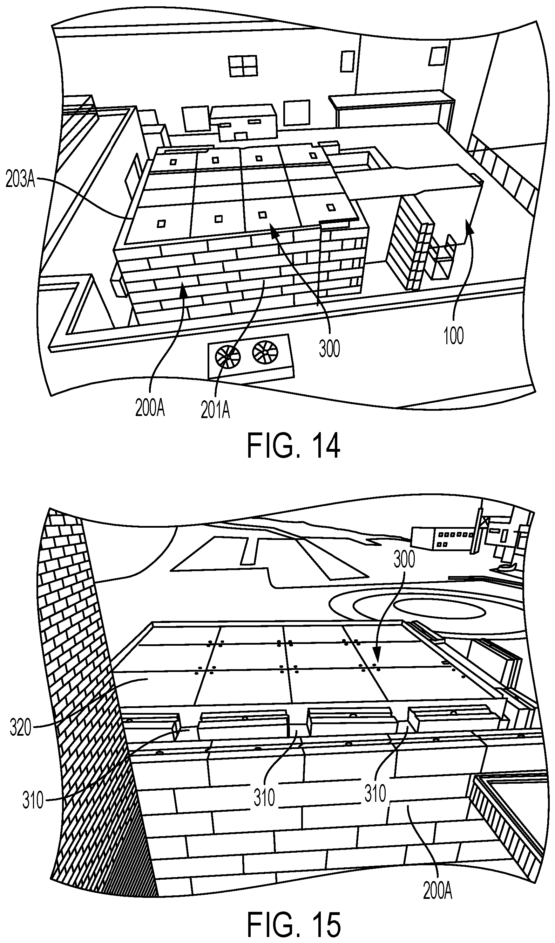

In other embodiments, as illustrated for example in FIG. 14, the cap shielding 300 is installed on top of the external shielding 200A and is configured to cover substantially the entire footprint of the external shielding 200A. In such configurations, the cap shielding 300 provides more comprehensive vertical shielding coverage, thereby further reducing the potential for radiation exposure in elevated surrounding areas. The extent of coverage provided by the cap shielding 300 may be selected based on site-specific factors, including but not limited to the height and proximity of adjacent structures, occupancy considerations, regulatory requirements, and the radiation output characteristics of the medical treatment facility.

The use of cap shielding 300 in combination with the external shielding 200A enables the portable radiation oncology system to maintain regulatory compliance and operational safety across a wide range of deployment environments, including sites with vertical exposure concerns, without reliance on permanent overhead shielding structures.

FIG. 15 depicts a top perspective view of the external shielding 200A and the cap shielding 300 during a construction or installation phase, illustrating placement of the cap shielding 300 configured to maintain radiation shielding continuity across an upper region of the shielding structure. In some embodiments, the cap shielding 300 is installed by first positioning two or more supporting frames 310 on top of the external shielding 200A. The supporting frames 310 are configured to provide sufficient structural strength to support the weight of a roof 320 and any associated shielding materials, while maintaining alignment and stability during installation and operation. As shown, the supporting frames 310 extend laterally across the top of the external shielding walls 200A. Each of the supporting frames 310 is supported along its edges by the upper portions of the external shielding walls 200A.

In some embodiments, visible alignment markings on an upper surface of the roof 320 facilitate controlled placement and verification of proper coverage and positional accuracy relative to the external shielding walls 200A.

After the roof 320 is installed on the supporting frames 310, any portions of the external shielding walls 200A not overlain by the supporting frames 310 can be extended upward to substantially align with the roof 320. Seals can be applied along the interfaces between the cap shielding 300 and the external shielding walls 200A so that, together, they define a substantially continuous shielding barrier. In this arrangement, the cap shielding 300 closes an otherwise open upper boundary of the external shielding 200A, thereby reducing or eliminating radiation leakage paths that could otherwise exist at the top of the shielding structure.

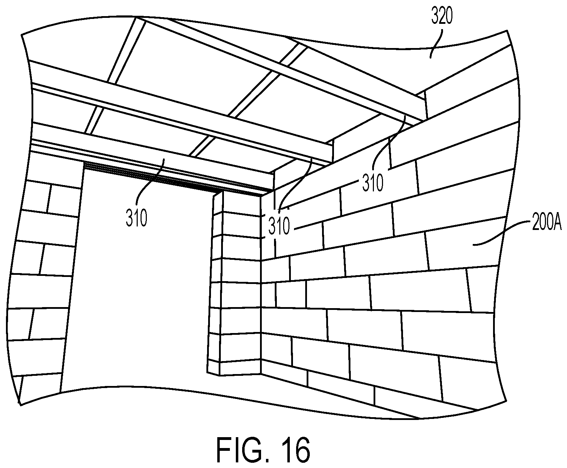

FIG. 16 depicts an internal view of the external shielding 200A with cap shielding 300 installed in a partially complete state during construction or installation with individual shielding blocks visible. In this view, the roof 320 of the cap shielding 300 is shown forming an overhead shielding component that extends across the interior space and is supported by supporting frames 310, which in turn is supported by upper regions of the external shielding walls 200A. The cap shielding 300 thereby defines an upper shielding boundary that cooperates with the external shielding 200A to form a substantially continuous radiation shielding barrier.

In some embodiments, the supporting frames 310 are formed from structural steel members selected to provide sufficient load-bearing capacity, rigidity, and resistance to deformation under the combined weight of the roof 320 and associated shielding materials. Suitable materials for the supporting frames 310 may include carbon structural steel, such as ASTM A36 steel, ASTM A572 high-strength low-alloy steel, or equivalent structural steel grades. In some embodiments, the supporting frames 310 comprises steel I-beams, wide-flange beams, H-beams, box beams, or tubular steel members arranged to span between opposing portions of the external shielding 200A. By way of non-limiting example, individual beams of the supporting frames 310 may have nominal depths in the range of approximately 8 inches (20.32 cm) to 24 inches (60.96 cm), flange widths in the range of approximately 6 inches (15.24 cm) to 12 inches (30.48 cm), and wall or flange thicknesses selected based on anticipated loads and span lengths.

In some embodiments, the roof 320 is formed from shielding materials selected to attenuate upwardly directed radiation leakage or scatter while also providing structural integrity and durability. Suitable materials for the roof 320 may include sheet steel, layered steel panels, steel-concrete composite panels, reinforced concrete panels, or combinations thereof. In some embodiments, the roof 320 comprises steel plates having a thickness in the range of approximately 1 inch to 8 inches (2.54 - 20.32 cm), optionally layered or combined with additional shielding materials to achieve a desired radiation level. In some embodiments, the roof 320 comprises modular panels having lateral dimensions in the range of approximately 8 feet by 10 feet, thereby facilitating transportation, handling, and on-site assembly. The modular roof panels may be supported directly on the supporting frames 310 and mechanically fastened, welded, or otherwise secured to the frame to form a stable overhead shielding structure. In some embodiments, the roof 320 is configured to be removable and reusable, allowing the cap shielding 300 to be adapted for different deployment sites and shielding requirements.

Once the supporting frames 310 are secured in place, roof 320 is positioned on top of the supporting frames 310 to form an overhead shielding structure. In some embodiments, the roof 320 is formed from sheet steel, layered steel panels, concrete panels, or other suitable shielding materials capable of attenuating upwardly directed radiation leakage or scatter. The roof 320 may be provided as a single continuous panel or as a plurality of modular sections that are assembled on site, thereby facilitating transportation, handling, and installation of the cap shielding 300.

In some embodiments, the modular configuration of the roof 320 allows individual panels or sections to be selectively installed, removed, or replaced based on site-specific shielding requirements, structural constraints, or regulatory considerations. The roof 320 may be mechanically fastened, welded, or otherwise secured to the supporting frames 310 to ensure proper positioning and structural integrity. Collectively, the supporting frames 310 and the roof 320 cooperate to define the cap shielding 300 as a removable, reusable, and adaptable overhead shielding structure that enhances radiation protection in environments where elevated surrounding areas or structures are present, while maintaining the portability of the overall radiation oncology system.

In the present embodiment, shielding below the unit 100 is not required for lateral barriers and for the rear of the unit 100.

The x-ray radiation generated by the portable unit 100 is typically 6 MV. It is typically when about 10 MV that pair production is achieved and elements become radioactive. As concrete is a low Z-element, even at high energy levels, advantageously no radioactive material is created. Accordingly, all external shielding barriers 200 will not be contaminated after using. In some embodiments, external shielding barriers 200 can be removed from site after use and can be reused.

A full radiation survey can be conducted around unit 100 after installation. Any areas that exceed limits for unrestricted areas will be marked as restricted areas. These areas may not be occupied by members of the public, and any professionals working in these areas may be permitted based on training and being equipped with the appropriate monitoring badge (personal monitor). Areas that have no occupancy may require little to no shielding. These areas can preferably be treated as restricted and access to these areas may be limited. Fences and appropriate signage may be required. These areas are preferably monitored closely by the staff and security.

Advantages of the portable radiation oncology system 10 described herein, compared to existing mobile radiation oncology coach systems formed by a tractor and trailer tandem, can be categorized in its design flexibility, operational efficiency, and patient accessibility.

First and foremost, this system 10 allows for the construction of a high ceiling, which is a significant design improvement over traditional mobile system. Existing mobile radiation oncology systems, particularly those built with a tractor-trailer configuration, often face spatial constraints, especially in terms of height. The ability to build a high ceiling within the portable radiation oncology system 10 provides more room for both medical equipment and personnel to operate comfortably, improving overall workflow and efficiency during treatment sessions.

Another major advantage is that once the portable radiation oncology system 10 is placed on site, it eliminates the need for stairs or patient lifts for patient access. In many mobile radiation oncology coach systems, patients often require assistance to board the vehicle, usually through the use of stairs or mechanical lifts, which can be difficult for patients with mobility challenges. The innovative design of this system 10 allows for easier, more direct access, making the system more inclusive, and reducing the logistical challenges typically faced when transporting patients in and out of the treatment area. In some embodiments, the portable radiation oncology system 10 is as low as approximately 12 inches (20.08 cm) from the ground. In some embodiments, the portable radiation oncology system 10 does not require external shielding as a result of the internal shielding and/or the lack of direct line of sight from the treatment area to the outside of the system.

The system 10 is also equipped with relocatable external shielding 200, which enhances both safety and flexibility. In contrast, traditional mobile systems often have permanent shielding that can limit the ability to reposition the system or adjust its layout for different site conditions. With the relocatable, movable or portable shielding in this new system, the external barriers can be adjusted or repositioned as needed, allowing for better adaptation to varying environmental and operational needs, such as local regulations, site configurations, or temporary treatment requirements.

In addition, due to the locations of walls 207, 208 (or 207A, 208A), the external shielding 200 (or 200A) is configured to cover three entire sides (i.e., rear side, left side, right side) of the treatment area 150. In addition, it is not necessary to have extra side barriers located near the vestibule 140 to prevent or reduce any potential radiation leaked through the pocket door 142 or the surrounding of the pocket door 142.

Lastly, the system 10 allows for a larger footprint, with the ability to achieve a width of 16 feet versus the more common 14 feet. This extra space provides significant advantages in terms of accommodating more equipment, expanding the treatment areas, and improving the comfort of both medical staff and patients. A larger footprint contributes to a more spacious and efficient operational environment, potentially increasing the overall effectiveness of the radiation oncology services provided.

It is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting.

As such, those skilled in the art will appreciate that the conception, upon which this disclosure is based, may readily be utilized as a basis for the design of other structures, methods and systems for carrying out the several purposes of the present invention. It is important, therefore, that the invention be regarded as including equivalent constructions/processes to those described herein insofar as they do not depart from the scope of the present invention as defined by the claims.

For example, the specific sequence of any described component and/or process may be altered. For example, certain processes are conducted in parallel or independent, with other processes, to the extent that the processes are not dependent upon each other. Other alterations or modifications of the above components and/or processes are also contemplated provided they fall within the scope of the claims. For example, further insubstantial changes to the components, systems and/or processes are also considered within the scope of the processes described herein.

The detailed description, for purposes of explanation, used specific nomenclature to provide a thorough understanding of the invention. However, it will be apparent to one skilled in the art that specific details are not required in order to practice the invention. Thus, detailed descriptions of specific embodiments of the invention are presented for purposes of illustration and description. They are not intended to be exhaustive or to limit the invention to the precise forms disclosed. Modifications and variations of the above detailed description are possible within the scope of the invention as defined by the claims.

## Claims

1. A portable radiation oncology system comprising:
a portable unit (100) configured to be placed on a level area, the portable unit including a control console area (130) and a treatment area (150), the treatment area being equipped with a medical treatment facility (152) that can emit radiation, the treatment area having a rear side, a left side, a right side opposing the left side, and a front side opposing the rear side and facing to the control console area;
internal shielding (148a) disposed between the control console area and the treatment area; and
external shielding (200) provided at a predetermined location that covers the entire rear side, left side, and right side of the treatment area.

2. The portable radiation oncology system of claim 1, wherein said portable unit is configured to be movable from a tractor to the level area by one or more lifts.

3. The portable radiation oncology system of claim 1 or 2, wherein said internal shielding comprises interlocked lead bricks, and optionally wherein a thickness of the interlocked lead bricks is 4 inches (10.16 cm) or wherein the interlocked lead bricks comprise a predetermined thickness to provide substantially effective shielding between the control console area and the treatment area.

4. The portable radiation oncology system of any preceding claim, further comprising:
a vestibule area (140) located between the control console area and the treatment room; and
second internal shielding (138a) provided between the vestibule area and the control console area.

5. The portable radiation oncology system of claim 4, wherein the second internal shielding comprises additional interlocked lead bricks comprising a second thickness to provide substantially effective shielding between the control console area and the vestibule area, and optionally wherein a thickness of the additional interlocked lead bricks is 2 inches (5.08 cm).

6. The portable radiation oncology system of claim 4, further comprising:
a first door configured and providing access between the treatment area and the vestibule area, the first door including first shielding; and
a second door configured and providing access between the vestibule area and the control console area, the second door is further configured to be installed on an opposite side of said trailer, preventing a direct line of sight between the treatment area and the control console area.

7. The portable radiation oncology system of claim 4, wherein the external shielding terminates approximately at the vestibule area.

8. The portable radiation oncology system of any preceding claim, further comprising:
a first door (142) configured and providing access between the treatment area and the control console area, the first door including first supplemental shielding, and optionally wherein the first door is further configured to be constructed and positioned to prevent a direct line of sight between the treatment area and the control console area.

9. The portable radiation oncology system of any preceding claim, wherein the medical treatment facility includes a medical linear particle accelerator, LINAC.

10. The portable radiation oncology system of any preceding claim, wherein the external shielding comprises a plurality of barriers (200), and optionally wherein the plurality of barriers comprise concrete and/or wherein the plurality of barriers forming two pieces not connected to each other.

11. The portable radiation oncology system of any preceding claim, further comprising a cap shielding (300) positioned above at least a portion of the external shielding to attenuate upwardly directed radiation leakage and/or scatter from the treatment area, wherein the cap shielding is supported by the external shielding and defines an overhead shielding structure above the treatment area, and optionally wherein the cap shielding covers less than an entire footprint defined by the external shielding to provide selective shielding of one or more regions above the treatment area, or wherein the cap shielding covers substantially an entire footprint defined by the external shielding.

12. The portable radiation oncology system of claim 11, wherein the cap shielding comprises a plurality of modular sections configured to be assembled on-site and disassembled for transportation and optionally wherein the cap shielding is removable and reusable for deployment at multiple sites.

13. The portable radiation oncology system of claim 11, wherein the cap shielding comprises two or more load-bearing supporting frames (310) disposed on top of the external shielding, and a roof portion (320) supported by the supporting frames, and optionally wherein the supporting frames comprise structural beams arranged to span between opposing portions of the external shielding.

14. A portable radiation oncology system comprising:
a portable unit (100) configured to be placed on a level area, the portable unit including:
a control console area (130), the control console area having a rear side, a left side, a right side opposing the left side, and a front side opposing the rear side;
a treatment area (150) equipped with a medical treatment facility (152) that can emit radiation;
first internal shielding (148a) disposed between the control console area and the treatment area;
a vestibule area (140) located between the control console area and the treatment room;
second internal shielding (138a) provided between the vestibule area and the control console area;
a first door (142) configured and providing access between the treatment area and the vestibule area, the first door including first shielding; and
a second door (136) configured and providing access between the vestibule area and the control console area, the second door configured to be installed on an opposite side of said first door, preventing a direct line of sight between the treatment area and the control console area.

15. The portable radiation oncology system of claim 14, wherein the second door is configured to be unshielded.

16. The portable radiation oncology system of claim 14 or 15, wherein an interior floor of the portable unit is less than 1.5 feet (45.72 cm) higher than a ground level around the portable unit.

17. The portable radiation oncology system of claim 14, 15 or 16, wherein the portable unit is configured to be detached from a flatbed trailer and placed on the level area in a clinical mode.

18. The portable radiation oncology system of any of claims 14 to 17, wherein the portable unit includes at least one slide-out section configured to increase an interior width of the portable unit when in a clinical mode.

19. The portable radiation oncology system of any of claims 14 to 18, wherein the medical treatment facility comprises a medical linear accelerator configured to emit radiation for radiotherapy.

20. The portable radiation oncology system of any of claims 14 to 19, wherein the first door comprises a sliding door.

21. The portable radiation oncology system of any of claims 14 to 20, wherein the first shielding comprises lead.

22. A method of installing a portable radiation oncology system comprising:
placing a portable unit (100) on a level area, the portable unit including a control console area (130) and a treatment area (150), the treatment area being equipped with a medical treatment facility that can emit radiation.
disposing an internal shielding (148a) between the control console area and the treatment area;
installing an external shielding (200) at a predetermined location that covers the entire rear side, left side, and right side of the treatment area; and
installing a cap shielding (300) above at least a portion of the external shielding to attenuate upwardly directed radiation leakage and/or scatter from the treatment area.

23. The method of claim 22, wherein the step of installing a cap shielding above at least a portion of the external shielding comprising:
disposing two or more load-bearing supporting frames (310) on top of the external shielding;
installing a roof portion (320) on the supporting frames; and
extending upward a portion of the external shielding not overlain by the supporting frames to substantially align with the roof portion.

24. The method of claim 22 or 23, further comprising:
estimating a total exposure using weighted dose rates at a plurality of locations within the portable unit;
determining, based at least in part on the measured dose rates, a console-area maximum dose rate; and
comparing the console-area maximum dose rate to a threshold dose rate to verify that the console-area maximum dose rate is less than the threshold dose rate.

25. The method of claim 22, 23 or 24, further comprising:
measuring dose rates at a plurality of locations outside of the portable unit.

26. The method of any of claims 22 to 25, wherein the step of placing the portable unit on the level area comprises detaching the portable unit from a trailer and lowering the portable unit onto the level area using one or more lifts.

27. The method of any of claims 22 to 26, further comprising:
disposing a second internal shielding between a vestibule area and the control console area.

28. The method of any of claims 22 to 27, wherein the step of installing the external shielding comprises assembling a plurality of modular shielding panels around the treatment area, and optionally wherein the step of assembling the plurality of modular shielding panels comprises positioning the modular shielding panels on a ground support structure and securing adjacent modular shielding panels to one another using mechanical fasteners.

29. The method of any of claims 22 to 28, wherein the cap shielding extends over at least a rear portion of the treatment area.

30. The method of any of claims 22 to 29, further comprising:
verifying radiation leakage and/or scatter after installing the external shielding and the cap shielding using one or more radiation detectors positioned outside the portable unit, and optionally wherein the step of verifying radiation leakage and/or scatter comprises taking measurements at a plurality of distances from the external shielding and comparing the measurements to a predetermined dose-rate threshold.

31. The method of any of claims 22 to 30, wherein the step of installing the external shielding at the predetermined location comprises positioning the external shielding such that the external shielding is spaced from the portable unit by a predetermined standoff distance, and/or wherein the step of installing the external shielding comprises providing an opening in the external shielding for access to the portable unit while maintaining shielding coverage of the rear side, left side, and right side of the treatment area.
